# EUROPEAN PATENT APPLICATION

(11) **EP 2 586 483 A1**
(43) Date of publication of application: **01.05.2013**
(21) Application number: 12187829.2
(22) Date of filing: 09.10.2012
(51) Int. Cl.: A61M 25/09

(54) **Guidewire**

(30) Priority: 31.10.2011 JP 2011238602
(71) Applicant: ASAHI INTECC CO., LTD., Nagoya-shi, Aichi 463-0024 (JP)
(72) Inventor: Miyata, Naohiko c/o ASAHI INTECC CO., LTD., Nagoya-shi, Aichi 463-0024 (JP); Sakakura, Aki c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft

(57) **Abstract**

A guidewire (1, 11, 21, 101, 111) includes a core shaft (2) and a coil body (3, 13, 23, 33, 43) that covers the core shaft (2). The guidewire also includes a tip portion, an intermediate fixing portion, and a proximal-end fixing portion that fix the core shaft (2) and the coil body (3, 13, 23, 33, 43) to each other. The coil body (3, 13, 23, 33, 43) includes a single core wire formed of a tungsten wire and a single side wire formed of a stainless steel wire. The side wire is wound around the core wire so as to cover the outer periphery of the core wire.

## Description

### BACKGROUND OF THE INVENTION

### 1. Field of the Invention

The present invention relates to a guidewire.

### 2. Description of the Related Art

Various guidewires used to insert, for example, a medical device into a tube, such as a blood vessel, an alimentary canal, or a ureter, or a body tissue and guide the medical device to a target area have been proposed.

For example, Japanese Unexamined Patent Application Publication No. 2009-337 describes a guidewire including a distal coil and a proximal coil. The distal coil has a coil spring configuration formed of a plurality of stranded wires, each of which is formed by twisting wires together. The proximal coil has a coil spring configuration formed of a signal wire. With this structure, both flexibility and torque transmission performance of a distal end portion of the guidewire can be increased.

As another example, Japanese Unexamined Patent Application Publication No. 2002-306607 describes a guidewire including a coil wire which includes a first wire and a second wire. A distal end portion of the first wire is made of a radiopaque material and the remaining portion of the first wire is made of a radiolucent material. The second wire is entirely made of a radiopaque highly elastic material. Tungsten, which has a high flexural rigidity, is used as the material of the second wire. With this structure, both radiopacity and flexibility of the distal end portion of the guidewire can be increased.

### SUMMARY OF THE INVENTION

In the guidewire described in Japanese Unexamined Patent Application Publication No. 2009-337, since the guidewire includes the distal coil having a low flexural rigidity formed by winding a plurality of stranded wires, flexibility of the guidewire is increased. However, sufficient torque transmission performance cannot be obtained by such a highly flexible guidewire.

In the guidewire described in Japanese Unexamined Patent Application Publication No. 2002-306607, the coil wire contains tungsten. However, tungsten is a metal having poor metal solder wettability, and is therefore difficult to solder. In addition, tungsten has a high melting point, and is therefore difficult to weld to another member. Accordingly, it is difficult to reliably bond the second wire, which is made of tungsten, to the first wire and bond the coil wire, which contains tungsten, to a core wire. As a result, there is a risk that the coil wire will be separated from the core wire.

The present invention has been made in view of the above-described problems, and an object of the present invention is to provide a guidewire in which bonding strength between a coil containing tungsten, which has poor metal solder wettability, and a core shaft and torque transmission performance of the guidewire are increased.

According to an aspect of the present invention, a guidewire includes a core shaft and a coil body that covers the core shaft. The coil body is formed of a coil wire including a core wire formed of a tungsten wire and a side wire formed of a stainless steel wire that is wound around the core wire so as to cover an outer periphery of the core wire. The core shaft and the side wire are fixed to each other with metal solder.

In the guidewire according to the aspect, the coil wire of the coil body includes the tungsten wire, which has a high flexural rigidity. Therefore, the torque transmission performance of the coil body can be increased and the torque transmission performance of the guidewire can be increased accordingly. In addition, the coil wire of the coil body includes the core wire formed of the tungsten wire and the side wire formed of the stainless steel wire. Therefore, the tungsten wire (core wire), which has poor metal solder wettability, can be fixed to the core shaft with the stainless steel wire (side wire), which has good metal solder wettability, interposed therebetween. Therefore, the fixing strength between the coil body, which includes the tungsten wire, and the core shaft can be increased.

### BRIEF DESCRIPTION OF THE DRAWINGS

Fig. 1 illustrates the overall structure of a guidewire according to a first embodiment of the present invention.
Fig. 2 illustrates the overall structure of a guidewire according to a second embodiment of the present invention.
Fig. 3A illustrates the overall structure of a guidewire according to a third embodiment of the present invention.
Fig. 3B is an enlarged view of part IIIB in Fig. 3A.
Fig. 3C illustrates a modification of a coil wire that forms a coil body illustrated in Fig. 3B.
Fig. 4 illustrates the overall structure of a guidewire according to a modification of the present invention.
Fig. 5 illustrates the overall structure of a guidewire according to another modification of the present invention.

### DESCRIPTION OF THE PREFERRED EMBODIMENTS

Guidewires according to embodiments of the present invention will now be described with reference to the drawings.

### First Embodiment

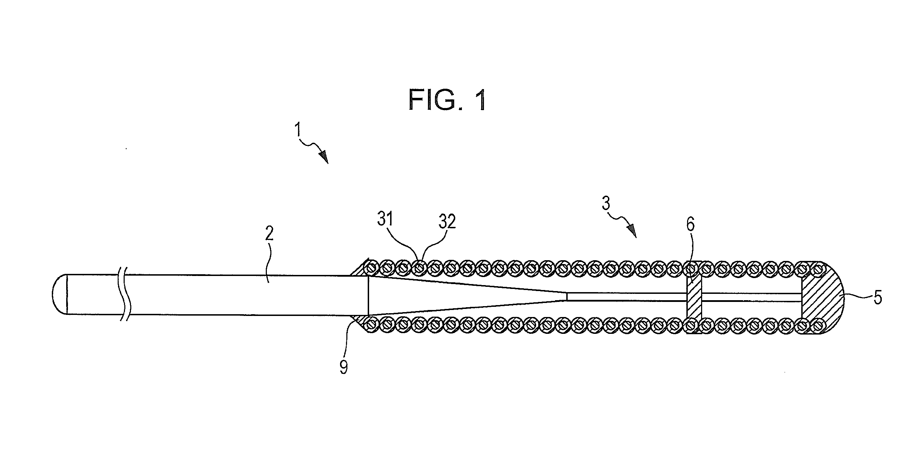

Fig. 1 illustrates the overall structure of a guidewire 1 according to a first embodiment of the present invention.

In Fig. 1, the left side will be described as "proximal-end side" and the right side will be described as "distal-end side" for convenience of explanation.

In addition, in Fig. 1, the length of the guidewire 1 is reduced and the guidewire 1 is schematically illustrated to facilitate understanding. The actual dimensions of the guidewire 1 are different from those in Fig. 1.

Referring to Fig. 1, the guidewire 1 includes a core shaft 2, a coil body 3 that covers a distal end portion of the core shaft 2, and a tip portion 5 that fixes the distal end of the coil body 3 and the distal end of the core shaft 2 to each other. The coil body 3 is fixed to the core shaft 2 by an intermediate fixing portion 6 at a position on the proximal-end side of the tip portion 5. The proximal end of the coil body 3 is fixed to the core shaft 2 by a proximal-end fixing portion 9.

The tip portion 5, the intermediate fixing portion 6, and the proximal-end fixing portion 9 that fix the core shaft 2 and the coil body 3 to each other are made of metal solder.

A coil wire of the coil body 3 includes a single core wire 31 formed of a tungsten wire and a single side wire 32 formed of a stainless steel wire. The side wire 32 is wound around the core wire 31 so as to surround the outer periphery of the core wire 31.

The tungsten wire included in the coil body 3 is a metal wire having a high flexural rigidity, and the stainless steel wire included in the coil body 3 is a metal wire whose flexural rigidity can be increased by subjecting the stainless steel wire to heat treatment or cold working. Although the tungsten wire is a metal wire having poor metal solder wettability, the stainless steel wire is a metal wire having good metal solder wettability.

Since the coil body 3 according to the present embodiment includes a tungsten wire, which has a high flexural rigidity, as the core wire 31 of the coil body 3, the torque transmission performance of the coil body 3 can be increased. As a result, the torque transmission performance of the guidewire 1 can be increased.

In addition, the tungsten wire (core wire 31), which has poor metal solder wettability, of the coil body 3 can be fixed to the core shaft 2 with the stainless steel wire (side wire 32), which has good metal solder wettability, interposed therebetween. Therefore, the fixing strength between the coil body 3, which includes the tungsten wire, and the core shaft 2 can be increased.

The metal solder used to fix the coil body 3 and the core shaft 2 to each other is preferably applied so as to fill the gap between the side wire 32 included in the coil wire of the coil body 3 and the core shaft 2.

In this case, the fixing strength between the coil body 3, which includes the tungsten wire, and the core shaft 2 can be further increased.

The side wire 32 may be densely wound such that portions of the side wire 32 that are adjacent to each other are in tight contact with each other. However although not illustrated, the side wire 32 is preferably loosely wound such that a gap (pitch) is provided between the portions of the side wire 32 that are adjacent to each other, the gap (pitch) being less than or equal to the diameter of the side wire 32. When a gap is provided between the adjacent portions of the side wire 32, the metal solder can easily flow into the gap between the adjacent portions of the side wire 32. As a result, the fixing strength between the coil body 3 and the core shaft 2 can be further increased.

Materials of the elements according to the present embodiment will now be explained. The material of the core shaft 2 is not particularly limited as long as the material is a metal having good metal solder wettability. For example, stainless steel (e.g., SUS304) or piano wire is suitable.

The material of the metal solder that forms the tip portion 5, the intermediate fixing portion 6, and the proximal-end fixing portion 9 that fix the core shaft 2 and the coil body 3 to each other may be, for example, a metal such as a Sn-Pb alloy, a Pb-Ag alloy, a Sn-Ag alloy, or a Au-Sn alloy.

To suppress a reduction in mechanical strength caused by a thermal effect on the core shaft 2 and the coil body 3, the metal solder preferably has a melting point that is lower than the melting points of the materials of the core shaft 2 and the coil body 3, as in the case where the above-mentioned materials are used. More preferably, to reliably prevent a reduction in mechanical strength caused by a thermal effect on the core shaft 2 and the coil body 3, the metal solder has a melting point that is lower than or equal to 450°C.

In particular, the material of the metal solder preferably contains gold as a main component. For example, the material of the metal solder may be a Au-Sn alloy. When a Au-Sn alloy is used as the metal solder, the stainless steel wire that forms the side wire 32 of the coil body 3 shows good metal solder wettability. In addition, Au-Sn alloys have a high mechanical strength, so that the fixing strength between the coil body 3 and the above-described core shaft 2 can be greatly increased. When the Au-Sn alloy has a melting point that is lower than or equal to 450°C, a reduction in mechanical strength caused by a thermal effect on the core shaft 2 and the coil body 3 can be prevented.

The stainless steel wire used as the side wire 32 included in the coil wire of the coil body 3 is preferably made of a metastable austenite stainless steel, such as SUS302, SUS304, or SUS316. When a metastable austenite stainless steel is subjected to wire drawing (cold working), the crystal structure thereof changes to (transformed into) a martensitic structure. As a result, the mechanical strength, such as flexural rigidity, hardness, and tensile strength, can be greatly increased. Therefore, when the side wire 32 included in the coil wire of the coil body 3 is formed of a metastable austenite stainless steel, the mechanical strength of the coil body 3 can be further increased and the torque transmission performance of the guidewire 1 can be greatly increased.

When the core shaft 2 and the coil body 3 are assembled together, flux is preferably applied to parts of the core shaft 2 and the coil body 3 that are to be fixed to each other. In this case, metal solder wettability of the core shaft 2 and the coil body 3 is increased and the fixing strength can be further increased.

Although not illustrated, the outer peripheral surface of the coil body 3 of the guidewire 1 may be coated with hydrophilic or hydrophobic lubricant. In such a case, slidability between the guidewire 1 and an object (such as an inner surface of a catheter or an inner wall of a blood vessel) that comes into contact with the guidewire 1 can be increased. As a result, the torque transmission performance of the guidewire 1 can be further increased.

The guidewire 1 of the present embodiment can be manufactured by the following method. First, the core shaft 2 having a distal end portion whose outer diameter is reduced is manufactured by grinding the outer peripheral surface of an end portion of a metal wire with a centerless grinding machine.

Next, a stainless steel wire is wound around a tungsten wire with a coil manufacturing device by using the tungsten wire as a coil core, and a heat treatment is performed at a temperature of 150°C to 500°C while the stainless steel wire is wound around the tungsten wire. Thus, the coil wire including the core wire 31 formed of a tungsten wire and the side wire 32 formed of a stainless steel wire is formed.

Next, the coil wire is wound around a coil core, such as a stainless steel wire or a copper wire, by using the coil manufacturing device and a heat treatment is performed at a temperature of 150°C to 500°C. Then, the coil core is pulled out. Thus, the coil body 3 is formed.

Next, the distal end of the core shaft 2 is inserted into the coil body 3 at the proximal end of the coil body 3, and the proximal end of the coil body 3 is fixed to the core shaft 2 with the metal solder by using a soldering iron or the like. Thus, the proximal-end fixing portion 9 is formed.

Next, the distal end of the coil body 3 is fixed to the distal end of the core shaft 2 with the metal solder by using a soldering iron or the like. Thus, the tip portion 5 is formed.

Lastly, the coil body 3 is fixed to the core shaft 2 with the metal solder at a position on the proximal-end side of the tip portion 5. Thus, the intermediate fixing portion 6 is formed and the guidewire 1 is completed.

The guidewire 1 may instead be formed by known methods other than the above-described method. For example, the coil body 3 may be formed by forming the side wire 32 in the shape of a coil in advance and inserting a tungsten wire that forms the core wire 31 through the coil-shaped side wire 32. In addition, a mold having a shape corresponding to the shape of the tip portion 5 may be prepared, and the tip portion 5 may be formed by setting the distal ends of the core shaft 2 and the coil body 3 to the mold and injecting the metal solder in a molten state into the mold.

### Second Embodiment

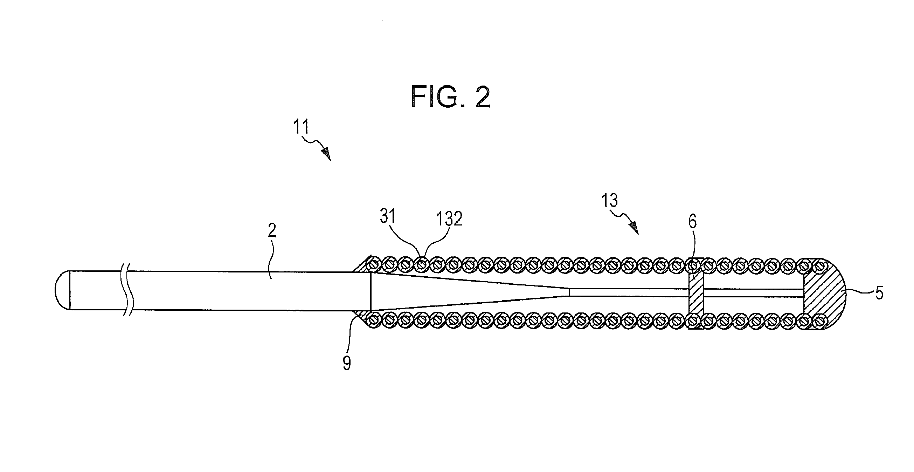

A guidewire 11 according to a second embodiment will now be described with reference to Fig. 2. Differences from the first embodiment will be mainly explained. In Fig. 2, components similar to those of the first embodiment are denoted by the same reference numerals.

In Fig. 2, the length of the guidewire 11 is reduced and the guidewire 11 is schematically illustrated to facilitate understanding. The actual dimensions of the guidewire 11 are different from those in Fig. 2.

Referring to Fig. 2, the structure of the second embodiment is similar to that of the first embodiment except that a coil wire of a coil body 13 included in the guidewire 11 includes a single core wire 31 formed of a tungsten wire and two side wires 132 formed of stainless steel wires. The side wires 132 are wound around the core wire 31 so as to cover the peripheral surface thereof.

Thus, in the guidewire 11 according to the second embodiment, the coil wire of the coil body 13 includes a single core wire 31 formed of a tungsten wire and two side wires 132 formed of stainless steel wires, the side wires 132 being wound around the core wire 31 so as to cover the peripheral surface thereof. Therefore, the mechanical strength, such as flexural rigidity, of the coil wire can be increased and the torque transmission performance of the guidewire 11 can be increased accordingly.

One of the two stainless steel wires that form the side wires 132 may have an outer diameter that is smaller than that of the other stainless steel wire. In such a case, the metal solder easily covers the outer periphery of the stainless steel wire having the smaller outer diameter. Thus, the fixing strength between the coil body 13 and the core shaft 2 can be increased while the mechanical strength of the coil wire of the coil body 13 is also increased.

### Third Embodiment

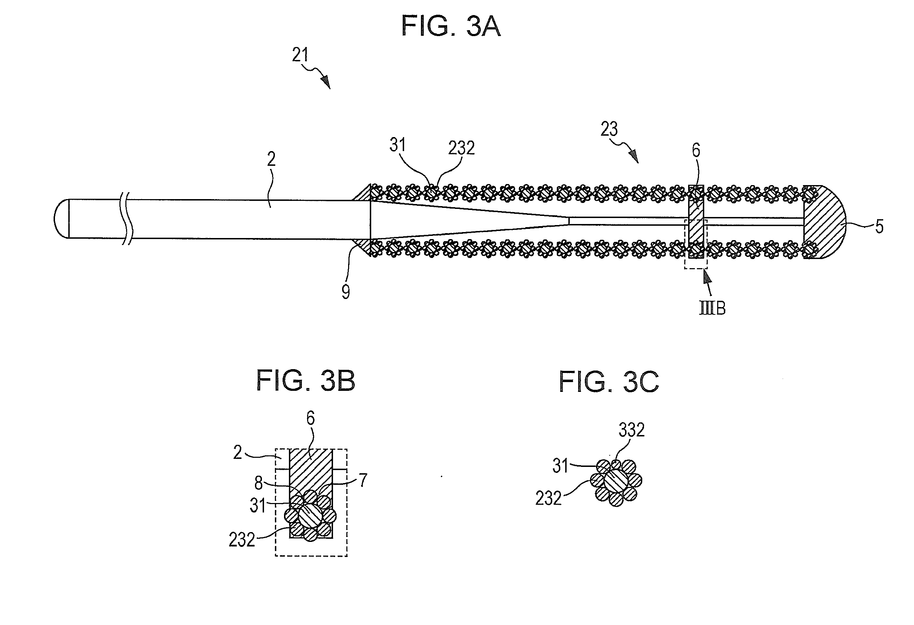

A guidewire 21 according to a third embodiment will now be described with reference to Figs. 3A, 3B, and 3C. Differences from the first and second embodiments will be mainly explained. In Figs. 3A, 3B, and 3C, components similar to those of the first and second embodiments are denoted by the same reference numerals.

In Fig. 3A, the length of the guidewire 21 is reduced and the guidewire 21 is schematically illustrated to facilitate understanding. The actual dimensions of the guidewire 21 are different from those in Fig. 3A. Fig. 3B is an enlarged view of part IIIB in Fig. 3A, and Fig. 3C illustrates a modification of a coil wire of a coil body illustrated in Fig. 3B.

Referring to Figs. 3A and 3B, the structure of the third embodiment is similar to that of the first embodiment except that a coil wire of a coil body 23 included in the guidewire 21 includes a single core wire 31 formed of a tungsten wire and eight side wires 232 formed of stainless steel wires. The side wires 232 are wound around the core wire 31 so as to cover the peripheral surface thereof.

Thus, in the guidewire 21 according to the third embodiment, the coil wire of the coil body 23 includes a single core wire 31 formed of a tungsten wire and eight side wires 232 formed of stainless steel wires, the side wires 232 being wound around the core wire 31 so as to cover the peripheral surface thereof. Therefore, the mechanical strength, such as flexural rigidity, of the coil wire can be increased and the torque transmission performance of the guidewire 21 can be greatly increased accordingly.

Since the coil wire of the coil body 23 includes eight side wires 232 formed of stainless steel wires, recessed portions 7 are formed between the outer peripheral surfaces of the side wires 232, that is, between the stainless steel wires. As illustrated in Fig. 3B, the metal solder that forms the intermediate fixing portion 6 fills the recessed portions 7. Therefore, the fixing strength between the core shaft 2 and the coil body 23, which is formed of a coil wire containing tungsten that has poor metal solder wettability, can be greatly increased.

When the number of stainless steel wires that form the side wires 232 included in the coil wire of the coil body 23 is increased, gaps 8 between the core wire 31 and the side wires 232 of the coil body 23 can be more easily filled with the metal solder.

Since the gaps 8 between the core wire 31 and the side wires 232 of the coil body 23 are filled with the metal solder, the outer periphery of the core wire 31, which is formed of a tungsten wire that has poor metal solder wettability, is covered by the metal solder and the side wires 232, which are formed of stainless steel wires that have good metal solder wettability. Therefore, the tungsten wire (core wire) can be reliably prevented from being separated from the coil body 23, and the coil body 23, which includes the tungsten wire, can be more reliably fixed to the core shaft 2.

Fig. 3C illustrates a modification of the coil wire of the coil body 23 illustrated in Fig. 3B. In this modification, the coil wire of the coil body 23 includes a single core wire 31 formed of a tungsten wire and eight side wires 232 and 332 formed of stainless steel wires, the side wires 232 and 332 being wound around the core wire 31 so as to cover the peripheral surface thereof. The eight side wires 232 and 332 include seven large-diameter side wires 232 and a single small-diameter side wire 332.

Since the side wires 232 and 332 include the large-diameter side wires 232 and the small-diameter side wire 332, the metal solder easily covers the outer periphery of the small-diameter side wire 332. Therefore, the fixing strength between the core shaft 2 and the coil body 23, which is formed of a coil wire containing tungsten that has poor metal solder wettability, can be greatly increased.

In this modification, seven large-diameter side wires 232 and a single small-diameter side wire 332 are provided. However, the present invention is not limited to this, and two or more small-diameter side wires 332 may, for example, be provided. Preferably, the number of small-diameter side wires 332 is smaller than the number of large-diameter side wires 232. When the number of small-diameter side wires 332 is smaller than the number of large-diameter side wires 232, sufficient strength of the coil wire of the coil body 23 can be ensured and the metal solder easily covers the outer periphery of the small-diameter side wire 332.

Although the number of side wires 232 and 332 is eight in the third embodiment illustrated in Figs. 3A and 3B and in the modification illustrated in Fig. 3C, the number of side wires 232 and 332 is not limited to this as long as the number is three or more.

The present invention is not limited to the above-described embodiments, and various modifications may be made by those skilled in the art within the technical idea of the present invention.

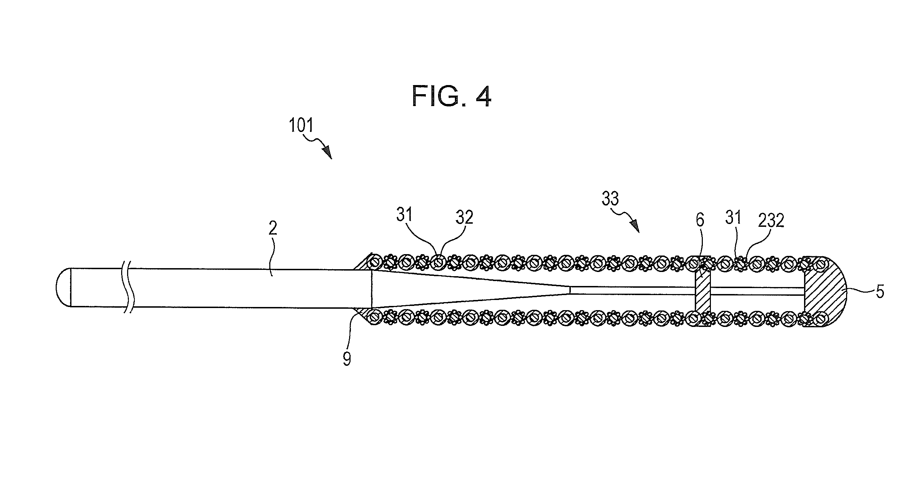

For example, a guidewire 101 according to a modification of the present invention is illustrated in Fig. 4. The guidewire 101 includes a coil body 33 formed of a coil wire identical to the coil wire of the coil body 3 included in the guidewire 1 of the first embodiment (the core wire 31 and the side wire 32) and a coil wire identical to the coil wire of the coil body 23 included in the guidewire 21 of the third embodiment (the core wire 31 and the side wires 232).

When the outer diameters of the core wire and the side wire in the coil wire of the coil body 3 according to the first embodiment are equal to those of the core wire and the side wires in the coil wire of the coil body 23 according to the third embodiment, the coil body 3 is more flexible than the coil body 23. Depending on the target of treatment (for example, a peripheral blood vessel of a coronary artery), the distal end portion of the guidewire may be required to be flexible while good torque transmission performance is ensured. When the coil wire of the coil body 3 and the coil wire of the coil body 23 are combined as in this modification, the flexibility can be increased while good torque transmission performance is ensured.

Thus, the coil wires of the coil body may be selected in accordance with the flexibility required of the guidewire according to the present invention. The coil wires of the coil body 33 may instead include, for example, two coil wires identical to the coil wire of the coil body 3 included in the guidewire 1 of the first embodiment and a single coil wire identical to the coil wire of the coil body 23 included in the guidewire 21 of the third embodiment. Thus, the numbers of the coil wires to be combined may be selected as appropriate.

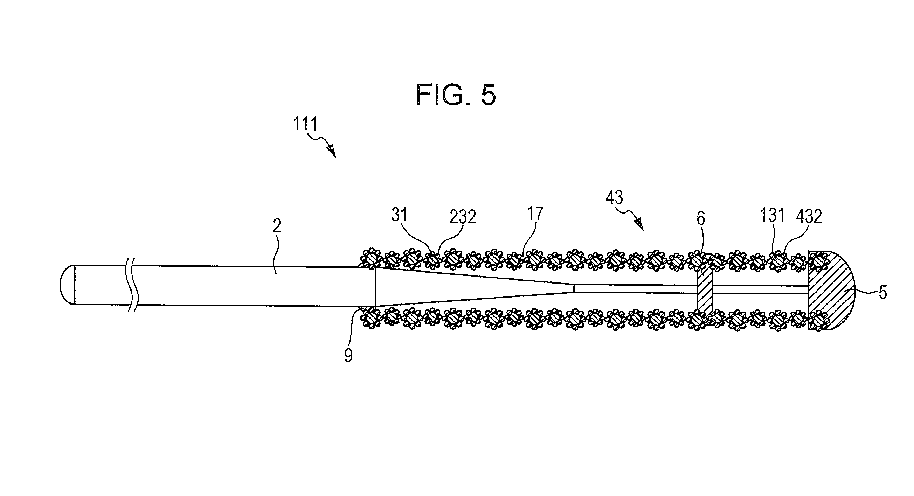

A guidewire 111 according to another modification is illustrated in Fig. 5. The guidewire 111 includes a coil body 43 formed of a coil wire identical to the coil wire of the coil body 23 included in the guidewire 21 of the third embodiment and a coil wire that has the same structure as the coil wire of the coil body 23 but includes a core wire 131 and side wires 432 having larger outer diameters than those of the core wire 31 and the side wires 232 included in the coil wire of the coil body 23. The coil body 43 is provided with recessed portions, which correspond to the recessed portions 7 illustrated in Fig. 3B, between the stainless steel wires that form the side wires 232 and 432 and recessed portions 17 between the large-diameter and small-diameter coil wires.

Since the coil body 43 of the guidewire 111 has the above-described structure, the mechanical strength, such as tensile strength and flexural rigidity, of the coil wires of the coil body 43 can be increased and the torque transmission performance of the guidewire 111 can be greatly increased accordingly. In addition, the coil wires of the coil body 43 have the recessed portions between the side wires 232 and 432 and the recessed portions 17 between the large-diameter and small-diameter coil wires. Therefore, the metal solder fills the recessed portions, so that the fixing strength between the core shaft 2 and the coil body 43, which is formed of a coil wire containing tungsten that has poor metal solder wettability, can be greatly increased.

## Claims

1. A guidewire (1, 11, 21, 101, 111) comprising:
a core shaft (2); and
a coil body (3, 13, 23, 33, 43) that covers the core shaft (2),
wherein the coil body (3, 13, 23, 33, 43) is formed of a coil wire including a core wire formed of a tungsten wire and a side wire formed of a stainless steel wire that is wound around the core wire so as to cover an outer periphery of the core wire, and
wherein the core shaft (2) and the side wire are fixed to each other with metal solder.

2. The guidewire according to claim 1,
wherein the number of the tungsten wires that form the core wire is one, and the number of the stainless steel wires that form the side wire is two or more.

3. The guidewire according to claim 1 or 2,
wherein a gap between the core shaft (2) and the coil body (3, 13, 23, 33, 43) is filled with the metal solder.

4. The guidewire according to one of claims 1 to 3,
wherein a gap between the core wire and the side wire is filled with the metal solder.
